# EUROPEAN PATENT SPECIFICATION

(11) **EP 2 296 759 B1**
(45) Date of publication and mention of the grant of the patent: **02.10.2019**
(21) Application number: 09749387.8
(22) Date of filing: 19.05.2009
(51) Int. Cl.: A61Q 11/00, A61Q 19/00, A61K 8/25, A61K 8/49, A61K 8/64, A61K 8/67, A61K 8/73, A61K 8/11, A23G 4/06, A23G 4/20

(54) **COOLING COMPOSITION**
KÜHLWIRKENDE ZUSAMMENSETZUNG
COMPOSITION RAFRAÎCHISSANTE

(30) Priority: 22.05.2008 US 55363
(43) Date of publication of application: 23.03.2011
(73) Proprietor: Givaudan SA, 1214 Vernier (CH)
(72) Inventor: FURRER, Stefan, Michael, Cincinnati Ohio 45215 (US); BELL, Karen, Ann, Loveland Ohio 45140 (US); CAMPANILE, Fabio, CH-8125 Zollikerberg (CH); DOORN, Aloysius, Lambertus, NL-3893 CB Zeewolde (NL); HAGEN, Joshua, Andrew, Cincinnati Ohio 45242 (US)
(74) Representative: Global Patents
(86) International application number: PCT/CH2009/000161
(87) International publication number: WO 2009/140783

(56) References cited:
- EP-A- 2 033 688
- WO-A-2004/045590
- WO-A-2007/019719
- WO-A-2009/051632
- US-A1- 2005 265 930
- US-A1- 2006 159 819
- US-A1- 2006 276 667
- US-A1- 2007 059 417
- US-A1- 2007 221 236

## Description

This disclosure relates to mixtures of cooling compounds for use in practical formulations.

Cooling compounds, that is, chemical compounds that impart a cooling sensation to the skin or mucous membranes of the body, are well known and widely used. Examples of successful compounds include (1R,2S,5R)-N-ethyl-2-isopropyl-5-methylcyclohexanecarboxamide and 2-isopropyl-N,2,3-trimethylbutanamide, commercialised respectively as WS-3™ and WS-23™. A recent highly effective cooling compound is (1R,2S,5R)-N-(4-(cyanomethyl)phenyl)-2-isopropyl-5-methylcyclohexane-carboxamide, Evercool™ 180 (hereinafter referred to as "180").

One problem with "180" has been the achievement of its maximum potential. Some cooling compounds do not dissolve well in solvents, which means that the quantity that can be added to some applications, and therefore the effect that can be perceived, is limited. Controlled release by means of encapsulation techniques has been suggested as a possible way of ameliorating this situation, but again there arises the problem of solubility with the solvents used in encapsulation. In addition, there can be problems with crystallisation of the compounds.

It has now been found that it is possible to considerably enhance the quantity of "180" that can be dissolved and therefore utilised in an application. There is therefore provided a method of providing a liquid cooling composition containing a primary cooling compound which is (1R,2S,5R)-N-(4-(cyanomethyl)phenyl)-2-isopropyl-5-methylcyclohexanecarboxamide, comprising the blending of (1R,2S,5R)-N-(4-(cyanomethyl)phenyl)-2-isopropyl-5-methylcyclohexanecarboxamide with at least one different secondary cooling compound selected from the group consisting of menthyl lactate, 2-isopropyl-N,2,3-trimethylbutanamide, and (1R,2S,5R)-2-isopropyl-5-methyl-N-(2-(pyridin-2-yl)ethyl)cyclo-hexanecarboxamide, and mixtures thereof, and at least one ingestible non-polar solvent for the primary cooling compound, selected from medium-chain triglyceride solvent (Miglyol™), peppermint oil, spearmint oil, triacetin, orange oil, lemon oil, lime oil, liquid flavours and menthol, the weight ratios of primary cooling compound: secondary cooling compound: solvent being 1:1.5-2.25:1.75-4.4.

There is additionally provided a method of incorporating at least one primary cooling compound in an application, comprising the blending of the primary cooling compound with at least one different secondary cooling compound and at least one ingestible non-polar solvent for the primary cooling compound, and the addition of the resulting mixture to the application.

By "liquid" is meant that the composition as formed is a liquid and remains thus, at least until incorporation into an end-product or into an entrapped form. It is a feature of the compositions that they can remain liquids for substantial periods of time at room temperature, varying from some hours to several weeks. In some cases, compositions can be held liquid at elevated temperature until processing. While crystallisation is not desirable in any circumstances, it is acceptable when it occurs when the composition has been incorporated into an end-product or into an entrapped form.

The composition has two different types of cooling compound, designated primary and secondary cooling compounds. By "primary" cooling compound is meant "180".

Secondary cooling compounds are selected from menthyl lactate, 2-isopropyl-N,2,3-trimethylbutanamide (WS-23™), (1R,2S,5R)-2-isopropyl-5-methyl-N-(2-(pyridin-2-yl)ethyl)cyclohexanecarboxamide (Evercool™ 190).

The non-polar solvents for use herein are ingestible liquids, that is, they are solvents that may be used in foodstuffs, beverages and orally-receivable medicinal products. They are capable of dissolving the primary cooling compound to at least some extent, preferably to the extent of at least 0.0005g/mL.

The solvents are selected from medium chain triglycerides (Mygliol™), peppermint oil, spearmint oil, triacetin, orange oil, lemon oil, lime oil, liquid flavors and menthol.

Particular compositions include:
(1R,2S,5R)-N-(4-(cyanomethyl)phenyl)-2-isopropyl-5-methylcyclohexanecarboxamide (Evercool™ 180) / menthyl lactate / Mygliol;
(1R,2S,5R)-N-(4-(cyanomethyl)phenyl)-2-isopropyl-5-methylcyclohexanecarboxamide (Evercool™ 180) / menthyl lactate / Triacetin;
(1R,2S,5R)-N-(4-(cyanomethyl)phenyl)-2-isopropyl-5-methylcyclohexanecarboxamide (Evercool™ 180) / 2-isopropyl-N,2,3-trimethylbutanamide (WS-23™) / Mygliol;
(1R,2S,5R)-N-(4-(cyanomethyl)phenyl)-2-isopropyl-5-methylcyclohexanecarboxamide (Evercool™ 180) / menthyl lactate / peppermint oil.
(1R,2S,5R)-N-(4-(cyanomethyl)phenyl)-2-isopropyl-5-methylcyclohexanecarboxamide (Evercool™ 180)/ (1R,2S,5R)-2-isopropyl-5-methyl-N-(2-(pyridin-2-yl)ethyl)cyclohexanecarboxamide (Evercool™ 190) / menthyl lactate/Triacetin.

In a particular embodiment, the ratios of primary cooling compound:secondary cooling compound: solvent are 1:1.8-2.2:2.5-3.5.

The liquid compositions of this disclosure are exceptionally robust, some exceptionally so, having good shelf stability and exhibiting little or no precipitation, even under conditions of variable temperature and humidity. They may be easily incorporated into all manner of foodstuffs, beverages and orally-receivable medicinal products.

These compositions may be used in products that are applied to mucous membranes such as oral mucosa, or to the skin, to give a cooling sensation. By "applying" is meant any form of bringing into contact, for example, oral ingestion, topical application or, in the case of tobacco products, inhalation. In the case of application to the skin, it may be, for example, by including the compound in a cream or salve. There is therefore also provided a method of providing a cooling sensation to mucous membranes or skin by applying thereto a product comprising an effective amount of a cooling composition as hereinabove described.

Products that are applied to the oral mucosa include, but are not limited to foodstuffs and beverages taken into the mouth and swallowed, and products taken for reasons other than their nutritional value, e.g. tablets, troches, mouthwash, throat sprays, dentifrices and chewing gums, which may be applied to the oral mucosa for the purpose of cleaning, freshening, healing, and/or deodorising.

Products that are applied to the skin include, but are not limited to perfumes, toiletries, cosmetic products such as lotions, oils, ointments and bathing agents, applicable to the skin of the human body, whether for medical or other reasons.

Particular examples of foodstuffs and beverages include, but are not limited to frozen confectionery such as ice creams and sorbets; desserts such as jelly and pudding; confectionery such as cakes, cookies, chocolates, and chewing gum; jams; candies; breads; tea beverages such as green tea, black tea, chamomile tea, mulberry leaf tea, Roobos tea, peppermint tea; soups; seasonings; instant beverages; snack foods and the like.

Further examples of products for topical application may include, but are not limited to, skin-care cosmetics such as cleansing tissues, talcum powders, face creams, lotions, tonics and gels; hand creams, hand- and body lotions, anticellulite/slimming creams and -lotions, lotions, balms, gels, sprays and creams; sunburn cosmetics including sunscreen lotions, balms, gels, sprays and creams; after sun lotions, sprays and creams; soaps, toothpicks, lip sticks, agents for bathing, deodorants and antiperspirants, face washing creams, massage creams, and the like,

Further examples of products that are applied to the oral mucosa may include, but are not limited to, oral care products such as toothpastes, tooth gels, tooth powders, tooth whitening products, dental floss, anti-plaque and anti-gingivitis compositions, compositions for treatment of nasal symptoms, and the like.

Thus, there is further provided an end-product selected from the group consisting of products that are applied to the oral mucosa and products that are applied to the skin, such as products for topical application, oral care products, nasal care products, toilet articles, ingestible products and chewing gum, and the like, which comprises a product base and an effective amount of at least one cooling composition comprising a blend of a primary cooling compound which is (1R,2S,5R)-N-(4-(cyanomethyl)phenyl)-2-isopropyl-5-methylcyclohexane-carboxamide, at least one different secondary cooling compound selected from the group consisting of menthyl lactate, 2-isopropyl-N,2,3-trimethyl-butanamide, and (1R,2S,5R)-2-isopropyl-5-methyl-N-(2-(pyridin-2-yl)ethyl)cyclohexanecarboxamide, and mixtures thereof, and at least one ingestible non-polar solvent for the primary cooling compound, selected from medium-chain triglyceride solvent (Miglyol™), peppermint oil, spearmint oil, triacetin, orange oil, lemon oil, lime oil, liquid flavours and menthol, the weight ratios of primary cooling compound: secondary cooling compound: solvent being 1:1.5-2.25:1.75-4.4.

The cooling compositions hereinabove described may also be blended with known natural sensate compounds, for example, jambu, galangal, galangal acetate, sanshool, capscacian, pepper and ginger, or other flavour and fragrance ingredients generally known to the person skilled in the art. Suitable examples of flavour and fragrance ingredients include alcohols, aldehydes, ketones, esters, ethers, acetates, nitriles, terpene hydrocarbons, nitrogenous or sulphurous heterocyclic compounds. Flavor and fragrance ingredients may be of natural or synthetic origin. Many of these are listed in reference texts such as the book by S. Arctander, Perfume and Flavor Chemicals, 1969, Montclair, New Jersey, USA. The cooling compositions may be employed in the products simply by directly mixing the composition with the product, or they may, in an earlier step, be entrapped in a suitable entrapment material. Suitable entrapment materials and means include, but are not limited to, polymer melts or hydrogels, capsules, microcapsules and nanocapsules, liposomes, film-formers and absorbents such as cyclic oligosaccharides (e.g., cyclodextrins). Alternatively, they may be added as a solution or emulsion in a suitable liquid, such liquids including alcohols or polyhydric alcohols (such as glycerine, propylene glycol) triacetin and medium chain triglycerides (Mygliol™), using emulsifers or dispersion stabilizers such as natural gums (e.g., gum Arabic) or low molecular-weight surfactants, such as glycerine fatty acid esters and saccharide fatty acid esters.

The capsules may be provided by any convenient method, for example, core-shell complex coacervation capsules, matrix type hydrogel capsules, and coated capsules. Examples of commercially-available entrapment materials useful with these compositions include the ULTRASEAL™, GRANUSEAL™ AND QPEARL™ products of Givaudan Flavors Corp., but any other similar technology may also be used.

In a particular embodiment, the compositions are used in a spray-dried form. Spray-drying is a technology well known to the art. Typically it involves the emulsification of the composition into water in the presence of a suitable emulsifier, and then spraying the emulsion into a heated air stream. The result is a fine particulate material. The emulsifier may be any suitable emulsifier known to the art, typical examples including gum arabic, food-modified starch, maltodextrin, gum ghatti, gelatine, sodium caseinate, whey protein, milk protein, sodium alginate and citrus pectin.

There is therefore provided a composition as hereinabove defined, in spray dried form.

When entrapped compositions are used in products, they may be the sole source of cooling compounds, or they may be augmented with other cooling compounds known in the art, e.g. menthol, menthone, isopulegol, N-ethyl p-menthanecarboxamide (WS-3 ™), N,2,3-trimethyl-2-isopropylbutanamide, (WS-23 ™), ethyl 2-(2-isopropyl-5methylcyclohexanecarboxamido)-acetate (WS-5 ™), menthyl lactate, menthone glycerine acetal (Frescolat® MGA), mono-menthyl succinate (Physcool®), mono-menthyl glutarate, O-menthyl glycerine (CoolAct® 10) and 2-sec-butylcyclohexanone (Freskomenthe®), menthane, camphor, pulegol, cineol, mint oil, peppermint oil, spearmint oil, eucalyptus oil, 3-l-menthoxypropane-1,2-diol, 3-l-menthoxy-2-methylpropane-1,2-diol, p-menthane-3,8-diol, 2-l-menthoxyethane-1-ol, 3-l-menthoxypropane-1-ol, 4-l-menthoxybutane-1-ol, and menthyl pyrrolidone carboxylic acid compounds sold under the commercial name Questice™. Further examples of cooling compounds can be found e.g. in WO 2005/049553 (e.g. 2-isopropyl-5-methyl-cyclohexanecarboxylic acid (4-cyanomethyl-phenyl)-amide and 2-isopropyl-5-methyl-cyclohexanecarboxylic acid (4-cyano-phenyl)-amide), WO2006/125334 (e.g. 4-[(2-isopropyl-5-methyl-cyclohexanecarbonyl)-amino]-benzamide, 3-[(2-isopropyl-5-methyl-cyclohexanecarbonyl)-amino]benzamide, and (2-isopropyl-5-methyl-N-(4-(4-methylpiperazine-1-carbonyl)phenyl)cyclohexanecarboxamide) and WO 2007/019719 (e.g. 2-isopropyl-5-methyl-cyclohexanecarboxylic acid pyridin-2-ylamide, and 2-isopropyl-5-methyl-cyclohexanecarboxylic acid (2-pyridin-2-yl-ethyl)-amide). These may be added conventionally in art-recognised quantities.

The compositions and methods are now further described with reference to the following non-limiting examples, which describe particular embodiments.

### Example 1:

Confirmation of suitable weight ratios of primary coolant (Evercool™ 180), secondary coolant (WS-23™) and solvent (Mygliol).

### General Procedure for preparation of the solutions:

Primary cooling agent was dissolved in molten secondary cooling agent and then diluted with solvent. The solution was allowed to cool to room temperature.

| **Primary Coolant** | **Secondary Coolant** | **Solvent** | **Soluble hot** | **Solution at RT** | **Time at room temperature stirred** | | | |
|---|---|---|---|---|---|---|---|---|
| **Evercool 180** | **WS-23** | **Mygliol** | | **standing 30min** | **3:0min** | **60min** | **90min** | **180min** |
| 0.25g | 0.25g | 0.5g | Yes | Yes | cryst. | cryst. | cryst. | cryst. |
| 0.25g | 0.25g | 0.6g | Yes | Yes | cryst. | cryst. | cryst. | cryst. |
| 0.25g | 0.25g | 0.7g | Yes | Yes | cryst. | cryst. | cryst. | cryst. |
| 0.25g | 0.25g | 0.8g | Yes | Yes | cryst. | cryst. | cryst. | cryst. |
| 0.25g | 0.25g | 0.9g | Yes | Yes | cryst. | cryst. | cryst. | cryst. |
| 0.25g | 0.25g | 1.0g | Yes | Yes | cryst. | cryst. | cryst. | cryst. |
| 0.28g | 0.42g | 0.5g | Yes | Yes | solution | solution | sl. cloudy | cryst. |
| 0.28g | 0.42g | 0.6g | Yes | Yes | solution | solution | sl. cloudy | cryst. |
| 0.28g | 0.42g | 0.7g | Yes | Yes | solution | solution | sl. cloudy | cryst. |
| 0.28g | 0.42g | 0.8g | Yes | Yes | solution | sl. cloudy | cryst. | cryst. |
| 0.28g | 0.42g | 0.9g | Yes | Yes | solution | sl. cloudy | cryst. | cryst. |
| 0.28g | 0.42g | 1.0g | Yes | Yes | cloudy | cryst. | cryst. | cryst. |
| 0.30g | 0.60g | 0.5g | Yes | Yes | solution | sl. cloudy | cryst. | cryst. |
| **0.30g** | **0.60g** | **0.6g** | **Yes** | **Yes** | **solution** | **solution** | **solution** | **solution** |
| **0.30g** | **0.60g** | **0.8g** | **Yes** | **Yes** | **solution** | **solution** | **solution** | **solution** |
| **0.30g** | **0.60g** | **0.9g** | **Yes** | **Yes** | **solution** | **solution** | **solution** | **solution** |
| 0.30g | 0.60g | 1.0g | Yes | Yes | solution | solution | sl. cloudy | cloudy |

The ratios primary coolant:secondary coolant: Mygliol of 1:1.5-2:2-3 are seen to be particularly effective.

### Example 2:

Primary coolant (Evercool™ 180), secondary coolant (WS-3™) and solvent (Mygliol) - Example of higher melting secondary coolant.

| Primary Coolant Evercool 180 | Secondary Coolant WS-3 | Solvent Mygliol | Soluble hot | |
|---|---|---|---|---|
| 1.0g | 2.0g | 3.0g | Yes | Crystallized at ∼ 50°C |

### Example 3:

Determination of optimal concentrations in orange oil (1x, Brazilian) as solvent, using WS-23™ as secondary coolant and Evercool™ 180 as primary coolant.

| | Concentration |
|---|---|
| Primary coolant: Evercool 180 | x% |
| Secondary coolant: WS-23 | 2x% |
| Solvent: Orange oil 1x Brazil | 100-3x% |

| time [min] | X% Primary Coolant (Secondary coolant = 2x%; solvent = 100-3x%) | | | | | | | | |
|---|---|---|---|---|---|---|---|---|---|
| | 7% | 9% | 11% | 13% | 14% | 16% | 17% | 20% | 22% |
| 5 | sol. | sol. | sol. | sol. | | sol. | sol. | sol. | sol. |
| 10 | sol. | sol. | sol. | sol. | | sol. | sol. | sol. | sol. |
| 15 | sol. | sol. | sol. | sol. | | sol. | sol. | sol. | sol. |
| 20 | sol. | sol. | sol. | sol. | | sol. | sol. | sol. | sol. |
| 30 | cloudy | sol. | sol. | sol. | | sol. | sol. | sol. | sol. |
| 40 | cryst. | cloudy | sol. | sol. | | sol. | sol. | sol. | sol. |
| 50 | cryst. | cryst. | cloudy | sol. | | sol. | sol. | sol. | sol. |
| 60 | cryst. | cryst. | cryst. | cloudy | | sol. | sol. | sol. | sol. |
| 70 | cryst. | cryst. | cryst. | cryst. | cloudy | sol. | sol. | sol. | sol. |
| 80 | cryst. | cryst. | cryst. | cryst. | cryst. | cloudy | sol. | sol. | sol. |
| 90 | cryst. | cryst. | cryst. | cryst. | cryst. | cloudy | sol. | sol. | sol. |
| 105 | cryst. | cryst. | cryst. | cryst. | cryst. | cryst. | sol. | sol. | sol. |
| 120 | cryst. | cryst. | cryst. | cryst. | cryst. | cryst. | cloudy | cloudy | sol. |
| 180 | cryst. | cryst. | cryst. | cryst. | cryst. | cryst. | cryst. | cryst. | cloudy |
| 210 | cryst. | cryst. | cryst. | cryst. | cryst. | cryst. | cryst. | cryst. | cryst. |

Solubility significantly improved compared to >1% of Evercool™ 180 in Orange oil (1x Brazilian) under regular conditions.

### Example 4:

Determination of optimal concentrations in triacetin as solvent, using WS-23™ as secondary coolant and Evercool™ 180 as primary coolant.

| | Concentration |
|---|---|
| Primary coolant: Evercool 180 | x% |
| Secondary coolant: WS-23 | 2x% |
| Solvent: Triacetin | 100-3x% |

| time [min] | x% Primary Coolant (Secondary coolant = 2x%; solvent = 100-3x%) | | | | | |
|---|---|---|---|---|---|---|
| | 7 % | 9% | 11% | 13% | 16% | 17% |
| 5 | sol. | sol. | sol. | sol. | sol. | sol. |
| 10 | sol. | sol. | sol. | sol. | sol. | sol. |
| 15 | sol. | sol. | sol. | sol. | sol. | sol. |
| 20 | sol. | sol. | sol. | sol. | sol. | sol. |
| 30 | sol. | sol. | sol. | sol. | sol. | sol. |
| 40 | sol. | sol. | sol. | sol. | sol. | sol. |
| 50 | sol. | sol. | sol. | sol. | sol. | sol. |
| 60 | sol. | sol. | sol. | sol. | sol. | sol. |
| 90 | sol. | sol. | sol. | sol. | sol. | sol. |
| 120 | sol. | sol. | sol. | sol. | sol. | sol. |
| 180 | sol. | sol. | sol. | sol. | sol. | sol. |
| 240 | sol. | sol. | sol. | sol. | sol. | sol. |
| 330 | sol. | sol. | cryst. | sol. | sol. | sol. |
| 420 | sol. | sol. | cryst. | sol. | sol. | sol. |
| 1440 | sol. | sol. | cryst. | sol. | sol. | cryst. |
| 1500 | cryst. | cryst. | cryst. | cryst. | cryst. | cryst. |

### Example 5:

Determination of optimal concentrations in menthol as solvent, using WS-23™ as secondary coolant and Evercool™ 180 as primary coolant.

| | Concentration |
|---|---|
| Primary coolant: Evercool 180 | x% |
| Secondary coolant: WS-23 | 2x% |
| Solvent: Menthol | 100-3x% |

| time [min] | x% Primary Coolant (Secondary coolant = 2x%; solvent = 100-3x%) | | | | | | |
|---|---|---|---|---|---|---|---|
| | 7 % | 9% | 11% | 13% | 14% | 16% | 17% |
| 15 | sol. | sol. | sol. | sol. | sol. | sol. | sol. |
| 30 | sol. | sol. | sol. | sol. | sol. | sol. | sol. |
| 45 | sol. | sol. | sol. | sol. | sol. | sol. | sol. |
| 60 | sol. | sol. | sol. | sol. | sol. | sol. | sol. |
| 75 | cryst. | sol. | sol. | sol. | sol. | sol. | sol. |
| 90 | cryst. | sol. | sol. | sol. | sol. | sol. | sol. |
| 165 | cryst. | sol. | sol. | sol. | sol. | sol. | sol. |
| 180 | cryst. | sol. | sol. | sol. | sol. | sol. | sol. |
| 240 | cryst. | sol. | sol. | sol. | sol. | sol. | sol. |
| 300 | cryst. | cryst. | sol. | sol. | sol. | sol. | sol. |
| 1380 | cryst. | cryst. | sol. | sol. | sol. | cloudy | sol. |
| 1560 | cryst. | cryst. | sol. | sol. | cloudy | cryst. | sol. |
| 1740 | cryst. | cryst. | sol. | sol. | cryst. | cryst. | cloudy |
| 2880 | cryst. | cryst. | sol. | cryst. | cryst. | cryst. | cryst. |
| 3180 | cryst. | cryst. | cloudy | cryst. | cryst. | cryst. | cryst. |

Solubility significantly improved, compared to 17% Evercool™ 180 soluble in menthol at 65°C under regular conditions

### Example 6:

Determination of optimal concentrations in Orange Oil lOx California as solvent, using WS-23™ as secondary coolant and Evercool 180 as primary coolant

| | Concentration |
|---|---|
| Primary coolant: Evercool 180 | x% |
| Secondary coolant: WS-23 | 2x% |
| Solvent: Orange Oil lOx California | 100-3x% |

| time [min] | x% Primary Coolant (Secondary coolant = 2x%; solvent = 100-3x%) | | | | |
|---|---|---|---|---|---|
| | 7% | 11% | 14% | 17% | 20% |
| 10 | sol. | sol. | sol. | sol. | sol. |
| 20 | sol. | sol. | sol. | sol. | sol. |
| 30 | sol. | sol. | sol. | sol. | sol. |
| 40 | sol. | sol. | sol. | sol. | sol. |
| 50 | sol. | sol. | sol. | sol. | sol. |
| 60 | sol. | sol. | sol. | sol. | sol. |
| 105 | sol. | sol. | sol. | sol. | sol. |
| 180 | sol. | sol. | sol. | sol. | sol. |
| 285 | sol. | cryst. | cryst. | cloudy | sol. |
| 345 | sol. | cryst. | cryst. | cryst. | cloudy |
| 360 | sol. | cryst. | cryst. | cryst. | cloudy |
| 1200 | cloudy | cryst. | cryst. | cryst. | cryst. |

### Example 7:

Determination of optimal concentrations in Peppermint oil as solvent, using WS-23™ as secondary coolant and Evercool™ 180 as primary coolant.

| | Concentration |
|---|---|
| Primary coolant: Evercool 180 | x% |
| Secondary coolant: WS-23 | 2x% |
| Solvent: Peppermint oil | 100-3x% |

| time [min] | x% Primary Coolant (Secondary coolant = 2x%; solvent = 100-3x%) | | | | | | | | | | |
|---|---|---|---|---|---|---|---|---|---|---|---|
| | 7% | 9% | 11% | 13% | 14% | 16% | 17% | 18% | 19% | 19% | 20% |
| 30 | sol. | sol. | sol. | sol. | sol. | sol. | sol. | sol. | sol. | sol. | sol. |
| 60 | sol. | sol. | sol. | sol. | sol. | sol. | sol. | sol. | sol. | sol. | sol. |
| 120 | sol. | sol. | sol. | sol. | sol. | sol. | sol. | sol. | sol. | sol. | sol. |
| 180 | sol. | sol. | sol. | sol. | sol. | sol. | sol. | sol. | sol. | sol. | sol. |
| 240 | sol. | sol. | sol. | sol. | sol. | sol. | sol. | sol. | sol. | sol. | sol. |
| 300 | sol. | sol. | sol. | sol. | sol. | sol. | sol. | sol. | sol. | sol. | sol. |
| 360 | sol. | sol. | sol. | sol. | sol. | sol. | sol. | sol. | sol. | sol. | sol. |
| 1440 | sol. | sol. | sol. | sol. | sol. | sol. | sol. | sol. | sol. | sol. | sol. |
| 2640 | sol. | sol. | sol. | sol. | sol. | sol. | sol. | sol. | sol. | sol. | cloudy |
| 2 weeks | sol. | sol. | sol. | sol. | sol. | sol. | sol. | sol. | cryst. | cryst. | cryst. |
| 6 weeks | sol. | sol. | sol. | sol. | sol. | sol. | sol. | sol. | cryst. | cryst. | cryst. |

Solubility significantly improved, compared to 12% Evercool™ 180 soluble in peppermint oil at room temperature under regular conditions.

### Example 8:

Encapsulation of a mixture of Menthyl Lactate (secondary coolant) and Evercool 180 (primary coolant) and Mygliol (solvent) in a non-cariogenic spray dry matrix:
The coolants may be solubilized in an oil phase before they are added to the spray dry matrix to form an emulsion. For this system, the 32g of menthyl lactate were melted above 60°C. 16g of Evercool 180 was added to this (2:1 weight ratio of menthyl lactate to Evercool 180) and heated to above 100°C until solubilized. 48g of hot Mygliol (above 100°C) was then added to this homogeneous solution at a 1:1 ratio. The solution then was held above 80°C to maintain solubility. This oil phase was then added to a gum arabic solution (36% solution in water, 400g gum arabic, 700g water) which was held at 60°C. This emulsion was then spray dried under normal conditions.

### Example 9:

Application of spray-dried mixture of Menthyl Lactate (secondary coolant) and Evercool™ 180 and comparison to references:

| | |
|---|---|
| Gum Base Solsona-T | 30 g |
| Sorbitol powdered | 50.6 g |
| Maltitol Syrup 85% | 9 g |
| Mannitol powdered | 5 g |
| Glycerin | 5 g |
| Acesulfame potassium (Ace-K™) | 0.09 g |
| Aspartame | 0.21 g |

Coolant mix spray dried (see amounts in table below)

| | | |
|---|---|---|
| 0.32g | Evercool 180 (spray dried with menthyl lactate; 3.2% load | Delivering 100ppm of Evercool 180 |
| 0.10g | Evercool 180 (spray dried without menthyl lactate; 10% load) [Reference] | Delivering 100ppm of Evercool 180 |
| 0.20g | Menthyl lactate (spray dried; 10% load) [Reference] | Delivering 200ppm of Menthyl lactate |

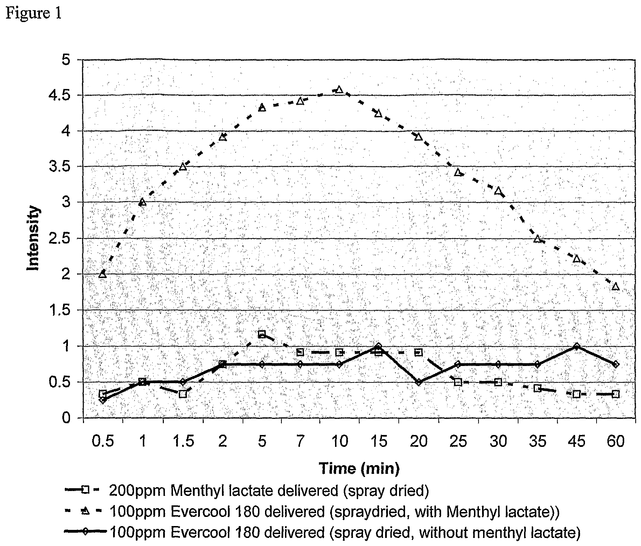

The gum base, and half of the sorbitol were mixed, maltitol syrup was added and then mixed with the gum mass. The rest of the powdered ingredients (rest of the sorbitol, mannitol, ace-K, aspartame) were added and mixed for about 1 minute, at which point glycerine was added and the gum mass was mixed for about 5 minutes, to form the blank chewing gum mass. The coolant mix was worked into the mass and a piece of the resulting gum (2 g) was chewed by a panelist for 20min and spat out. The cooling sensation was evaluated and its time intensity profile were recorded (see Figure 1).

### Example 10:

Encapsulation of a mixture of Menthyl Lactate (secondary coolant) and Evercool 180 (primary coolant) and Mygliol (solvent) in an encapsulation matrix:
The coolants must be solubilized in an oil phase before they are added to the spray dry matrix to form an emulsion. For this system, the 50g of menthyl lactate were melted above 60°C. 25g of Evercool 180 was added to this (2:1 weight ratio of menthyl lactate to Evercool 180) and heated to above 100°C until solubilized and added to 95g of hot Mygliol (above 75°C). This solution then was added to a hot (75°C) solution of 132g of Gelatin 100, 42g of Gelatin 0, 13g of Potato starch and 43g of Xylitol in 340g of Water and homogenized. The solution was kept stirring at 75°C during coating onto 100g of Gelatin 100 using a Wurster insert (Tₛₒₗᵤₜᵢₒₙ: 75 °C, P_{nozzle}: 2.5 bar, Tᵢₙₗₑₜ: 90 °C).

### Example 11:

Application of encapsulated mixture of Menthyl Lactate (secondary coolant) and Evercool™ 180 and comparison to references:

| | |
|---|---|
| Gum Base Solsona-T | 30 g |
| Sorbitol powdered | 50.6 g |
| Maltitol Syrup 85% | 9 g |
| Mannitol powdered | 5 g |
| Glycerin | 5 g |
| Acesulfame potassium (Ace-K™) | 0.09 g |
| Aspartame | 0.21 g |

Coolant mix encapsulated or spray dried (see amounts in table below)

| | | |
|---|---|---|
| 0.20g | Evercool 180 (encapsulated with menthyl lactate; 5% load | Delivering 100ppm of Evercool 180 |
| 0.20g | Menthyl lactate (spray dried; 10% load) [Reference] | Delivering 200ppm of Menthyl lactate |

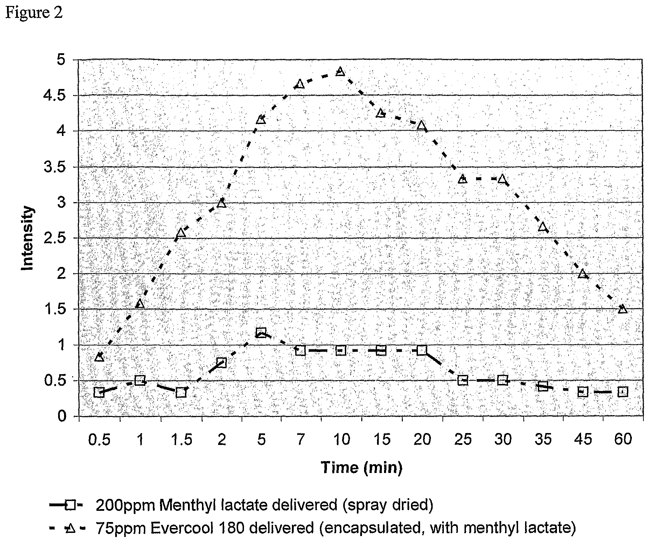

The gum base, and half of the sorbitol were mixed, maltitol syrup was added and then mixed with the gum mass. The rest of the powdered ingredients (rest of the sorbitol, mannitol, ace-K, aspartame) were added and mixed for about 1 minute, at which point glycerine was added and the gum mass was mixed for about 5 minutes, to form the blank chewing gum mass. The coolant mix was worked into the mass and a piece of the resulting gum (2 g) was chewed by a panelist for 20min and spat out. The cooling sensation was evaluated and its time intensity profile were recorded (see Figure 2).

It will be understood that the embodiments described herein are merely exemplary and that variations and modifications can be made by one skilled in the art without departing from the scope of the invention. It should be understood that the embodiments described above are not only in the alternative, but can be combined.

## Claims

1. A method of providing a liquid cooling composition containing a primary cooling compound which is (1R,2S,5R)-N-(4-(cyanomethyl)phenyl)-2-isopropyl-5-methylcyclohexane-carboxamide, comprising the blending of (1R,2S,5R)-N-(4-(cyanomethyl)phenyl)-2-isopropyl-5-methylcyclohexanecarboxamide with at least one different secondary cooling compound selected from the group consisting of menthyl lactate, 2-isopropyl-N,2,3-trimethylbutanamide, and (1R,2S,5R)-2-isopropyl-5-methyl-N-(2-(pyridin-2-yl)ethyl)cyclo-hexanecarboxamide, and mixtures thereof, and at least one ingestible non-polar solvent for the primary cooling compound, selected from medium-chain triglyceride, peppermint oil, spearmint oil, triacetin, orange oil, lemon oil, lime oil, liquid flavours and menthol, the weight ratios of primary cooling compound: secondary cooling compound: solvent being 1:1.5-2.25:1.75-4.4.

2. A method according to claim 1, in which the weight ratios of primary cooling compound: secondary cooling compound: solvent are 1:1.8-2.2:2.5-3.5.

3. A method according to claim 1, in which the non-polar solvent is capable of dissolving the primary cooling compound to the extent of at least 0.0005g/mL.

4. A method according to claim 1, in which the primary cooling compound/secondary cooling compound/solvent combination is selected from the group consisting of:
(1R,2S,5R)-N-(4-(cyanomethyl)phenyl)-2-isopropyl-5-methylcyclohexanecarboxamide / menthyl lactate / medium-chain triglyceride solvent;
(1R,2S,5R)-N-(4-(cyanomethyl)phenyl)-2-isopropyl-5-methylcyclohexanecarboxamide / menthyl lactate / Triacetin;
(1R,2S,5R)-N-(4-(cyanomethyl)phenyl)-2-isopropyl-5-methylcyclohexanecarboxamide / 2-isopropyl-N,2,3-trimethylbutanamide / medium-chain triglyceride solvent;
(1R,2S,5R)-N-(4-(cyanomethyl)phenyl)-2-isopropyl-5-methylcyclohexanecarboxamide / menthyl lactate / peppermint oil.; and
(1R,2S,5R)-N-(4-(cyanomethyl)phenyl)-2-isopropyl-5-methylcyclohexanecarboxamide)/ (1R,2S,5R)-2-isopropyl-5-methyl-N-(2-(pyridin-2-yl)ethyl)-cyclohexanecarboxamide / menthyl lactate/Triacetin.

5. A product adapted to be applied to at least one of the oral mucosa and the skin, which comprises a product base and an effective amount of a cooling composition comprising a blend of a primary cooling compound which is (1R,2S,5R)-N-(4-(cyanomethyl)phenyl)-2-isopropyl-5-methylcyclohexane-carboxamide, at least one different secondary cooling compound selected from the group consisting of menthyl lactate, 2-isopropyl-N,2,3-trimethyl-butanamide, and (1R,2S,5R)-2-isopropyl-5-methyl-N-(2-(pyridin-2-yl)ethyl)cyclo-hexanecarboxamide, and mixtures thereof, and at least one ingestible non-polar solvent for the primary cooling compound, selected from medium-chain triglyceride (Miglyol™), peppermint oil, spearmint oil, triacetin, orange oil, lemon oil, lime oil, liquid flavours and menthol, the weight ratios of primary cooling compound: secondary cooling compound: solvent being 1:1.5-2.25:1.75-4.4.

6. A product according to claim 5, in which the cooling composition is present in an entrapped form.

7. A product according to claim 6, in which the entrapped form is selected from the group consisting of polymer melts or hydrogels, capsules, microcapsules and nanocapsules, liposomes, film-formers and absorbents.

8. A product according to claim 6, in which the entrapped form is a spray-dried granulate.

## Patentansprüche

1. Verfahren zum Bereitstellen einer flüssigen kühlwirkenden Zusammensetzung, die eine primäre kühlwirkender Verbindung enthält, die (1R,2S,5R)-N-(4-(Cyanomethyl)phenyl)-2-isopropyl-5-methylcyclohexancarboxamid ist, das die Mischung von (1R,2S,5R)-N-(4-(Cyanomethyl)phenyl)-2-isopropyl-5-methylcyclohexancarboxamid mit mindestens einer anderen sekundären kühlwirkenden Verbindung umfasst ausgewählt aus der Gruppe bestehend aus Menthyllactat, 2-Isopropyl-N,2,3-trimethylbutanamid und (1R,2S,5R)-2-Isopropyl-5-methyl-N-(2-(pyridin-2-yl)ethyl)cyclohexancarboxamid und Mischungen davon und mindestens einem unverdaulichen nichtpolaren Lösungsmittel für die primäre kühlwirkende Verbindung ausgewählt unter Pfefferminzöl aus Triglycerid mittlerer Kette, Ährenminzeöl, Triacetin, Orangenöl, Zitronenöl, Limonenöl, flüssigen Geschmacksstoffen und Menthol, wobei die Gewichtsverhältnisse der primären kühlwirkenden Verbindung:sekundären kühlwirkenden Verbindung:Lösungsmittel 1:1,5-2,25:1,75-4,4 betragen.

2. Verfahren nach Anspruch 1, wobei die Gewichtsverhältnisse der primären kühlwirkenden Verbindung:sekundären kühlwirkenden Verbindung:Lösungsmittel 1:1,8-2,2:2,5-3,5 betragen.

3. Verfahren nach Anspruch 1, wobei das nichtpolare Lösungsmittel in der Lage ist, die primäre kühlwirkende Verbindung bis zu einem Ausmaß von mindestens 0,0005 g/ml zu lösen.

4. Verfahren nach Anspruch 1, wobei die primäre kühlwirkenden Verbindung/sekundäre kühlwirkenden Verbindung/Lösungsmittel-Kombination aus der Gruppe ausgewählt wird bestehend aus (1R,2S,5R)-N-(4-(Cyanomethyl)phenyl)-2-isopropyl-5-methylcyclohexancarboxamid/Menthyllactat/Lösungsmitte l aus Triglycerid mittlerer Kette;
(1R,2S,5R)-N-(4-(Cyanomethyl)phenyl)-2-isopropyl-5-methylcyclohexancarboxamid/Menthyllactat/Triacetin;
(1R,2S,5R)-N-(4-(Cyanomethyl)phenyl)-2-isopropyl-5-methylcyclohexancarboxamid/Isopropyl-N,2,3-trimethylbutanamid/Lösungsmittel- aus Triglycerid mittlerer Kette;
(1R,2S,5R)-N-(4-(Cyanomethyl)phenyl)-2-isopropyl-5-methylcyclohexancarboxamid/Mentyllactat/Pfefferminzöl ; und
(1R,2S,5R)-N-(4-(Cyanomethyl)phenyl)-2-isopropyl-5-methylcyclohexancarboxamid/(1R,2S,5R)-2-Isopropyl-5-methyl-N-(2-(pyridin-2-yl)ethyl)cyclohexancarboxamid/ Menthyllactat/Triacetin.

5. Produkt, das geeignet ist, auf mindestens eine von der Mundschleimhaut und der Haut aufgebracht zu werden, das eine Produktbase und eine wirksame Menge einer kühlwirkenden Zusammensetzung umfasst, die eine Mischung einer primären kühlwirkenden Verbindung umfasst, die (1R,2S,5R)-N-(4-(Cyanomethyl)phenyl)-2-isopropyl-5-methylcyclohexancarboxamid, mindestens eine andere sekundäre kühlwirkende Zusammensetzung ausgewählt aus der Gruppe bestehend aus Menthyllactat, 2-Isopropyl-N,2,3-trimethylbutanamid und (1R,2S,5R)-2-1sopropyl-5-methyl-N-(2-(pyridin-2-yl)ethyl)cyclo-hexancarboxamid und Mischungen davon und mindestens ein unverdauliches nichtpolares Lösungsmittel für die primäre kühlwirkende Verbindung ist ausgewählt unter Triglycerid mittlerer Kette (Miglyol^{WZ}), Pfefferminzöl, Ährenminzeöl, Triacetin, Orangenöl, Zitronenöl, Limonenöl, flüssigen Geschmacksstoffen und Menthol, wobei die Gewichtsverhältnisse der primären kühlwirkenden Verbindung:sekundären kühlwirkenden Verbindung:Lösungsmittel 1:1,5-2,25:1,75-4,4 betragen.

6. Produkt nach Anspruch 5, wobei die kühlwirkende Zusammensetzung in einer eingeschlossenen Form vorliegt.

7. Produkt nach Anspruch 6, wobei die eingeschlossene Form aus der Gruppe ausgewählt ist bestehend aus Polymerschmelzen oder Hydrogelen, Kapseln, Mikrokapseln und Nanokapseln, Liposomen, Filmbildnern und Absorptionsmitteln.

8. Produkt nach Anspruch 6, wobei die eingeschlossene Form ein sprühgetrocknetes Granulat ist.

## Revendications

1. Procédé de production d'une composition de refroidissement liquide contenant un composé de refroidissement primaire qui est le (1R,2S,5R)-N-(4-(cyanométhyl)phényl)-2-isopropyl-5-méthylcyclohexanecarboxamide, comprenant le mélange de (1R,2S,5R)-N-(4-(cyanométhyl)phényl)-2-isopropyl-5-méthylcyclohexanecarboxamide avec au moins un composé de refroidissement secondaire différent choisi dans le groupe constitué du lactate de menthyle, du 2-isopropyl-N,2,3-triméthylbutanamide et du (1R,2S,5R)-2-isopropyl-5-méthyl-N-(2-(pyridin-2-yl)éthyl)cyclohexanecarboxamide, et des mélanges de ceux-ci, et au moins un solvant non polaire ingérable pour le composé de refroidissement primaire, choisi parmi un triglycéride à chaîne moyenne, l'huile de menthe poivrée, l'huile de menthe verte, la triacétine, l'huile d'orange, l'huile de citron, l'huile de citron vert, des arômes liquides et le menthol, les rapports en poids composé de refroidissement primaire : composé de refroidissement secondaire : solvant étant 1:1,5-2,25:1,75-4,4.

2. Procédé selon la revendication 1, dans lequel les rapports en poids de composé de refroidissement primaire : composé de refroidissement secondaire : solvant sont 1:1,8-2,2:2,5-3,5.

3. Procédé selon la revendication 1, dans lequel le solvant non polaire est capable de dissoudre le composé de refroidissement primaire à un degré d'au moins 0,0005 g/ml.

4. Procédé selon la revendication 1, dans lequel la combinaison composé de refroidissement primaire / composé de refroidissement secondaire / solvant est choisie dans le groupe constitué de :
(1R,2S,5R)-N-(4-(cyanométhyl)phényl)-2-isopropyl-5-méthylcyclohexane-carboxamide / lactate de menthyle / solvant triglycéride à chaîne moyenne ;
(1R,2S,5R)-N-(4-(cyanométhyl)phényl)-2-isopropyl-5-méthylcyclohexane-carboxamide / lactate de menthyle / triacétine ;
(1R,2S,5R)-N-(4-(cyanométhyl)phényl)-2-isopropyl-5-méthylcyclohexane-carboxamide / 2-isopropyl-N,2,3-triméthylbutanamide / solvant triglycéride à chaîne moyenne ;
(1R,2S,5R)-N-(4-(cyanométhyl)phényl)-2-isopropyl-5-méthylcyclohexane-carboxamide / lactate de menthyle / huile de menthe poivrée ; et
(1R,2S,5R)-N-(4-(cyanométhyl)phényl)-2-isopropyl-5-méthylcyclohexane-carboxamide) / (1R,2S,5R)-2-isopropyl-5-méthyl-N-(2-(pyridin-2-yl)éthyl)-cyclohexanecarboxamide / lactate de menthyle / triacétine.

5. Produit adapté pour être appliqué sur au moins l'une parmi la muqueuse buccale et la peau, qui comprend une base de produit et une quantité efficace d'une composition de refroidissement comprenant un mélange d'un composé de refroidissement primaire qui est le (1R,2S,5R)-N-(4-(cyanométhyl)phényl)-2-isopropyl-5-méthylcyclohexane-carboxamide, au moins un composé de refroidissement secondaire différent choisi dans le groupe constitué du lactate de menthyle, du 2-isopropyl-N,2,3-triméthyl-butanamide et du (1R,2S,5R)-2-isopropyl-5-méthyl-N-(2-(pyridin-2-yl)éthyl)cyclohexanecarboxamide, et des mélanges de ceux-ci, et au moins un solvant non polaire ingérable pour le composé de refroidissement primaire, choisi parmi un triglycéride à chaîne moyenne (Miglyol™), l'huile de menthe poivrée, l'huile de menthe verte, la triacétine, l'huile d'orange, l'huile de citron, l'huile de citron vert, des arômes liquides et le menthol, les rapports en poids composé de refroidissement primaire : composé de refroidissement secondaire : solvant étant 1:1,5-2,25:1,75-4,4.

6. Produit selon la revendication 5, dans lequel la composition de refroidissement est présente sous une forme piégée.

7. Produit selon la revendication 6, dans lequel la forme piégée est choisie dans le groupe constitué de masses fondues ou d'hydrogels de polymère, de capsules, de microcapsules et de nanocapsules, de liposomes, d'agents filmogènes et d'absorbants.

8. Produit selon la revendication 6, dans lequel la forme piégée est un granulat séché par pulvérisation.
